# EUROPÄISCHE PATENTSCHRIFT

(11) **EP 1 704 864 B1**
(45) Veröffentlichungstag und Bekanntmachung des Hinweises auf die Patenterteilung: **23.05.2012**
(21) Anmeldenummer: 06012171.2
(22) Anmeldetag: 07.06.2000
(51) Int. Cl.: A61K 47/48, A61K 49/00, A61K 51/00, A61P 35/00

(54) **Injizierbare Arzneimittelzubereitung bestehend aus einem Zytostatikum und einer durch einen Spacer gebundenen Maleinimidgruppe.**
Injectable pharmaceutical preparation consisting of a cytostatic agent and a maleinimide linked by a spacer.
Préparation pharmaceutique injectable constituée d'un agent cytostatique et d'une fonction maléinimide liés par un espaceur.

(30) Priorität: 09.06.1999 DE 19926154
(43) Veröffentlichungstag der Anmeldung: 27.09.2006
(62) Teilanmeldung aus: 00945721.9
(73) Patentinhaber: KTB Tumorforschungs GmbH, 79106 Freiburg (DE)
(72) Erfinder: Kratz, Felix, 79106 Freiburg (DE)
(74) Vertreter: Dey, Michael

(56) Entgegenhaltungen:
- DE-A1- 19 636 889
- DE-A1- 19 923 168
- KRATZ F ET AL: "PREPARATION, CHARACTERIZATION AND IN VITRO EFFICACY OF ALBUMIN CONJUGATES OF DOXORUBICIN" BIOLOGICAL & PHARMACEUTICAL BULLETIN (OF JAPAN), PHARMACEUTICAL SOCIETY OF JAPAN, TOKYO, JP, Bd. 21, Nr. 1, Januar 1998 (1998-01), Seiten 56-61, XP000738275 ISSN: 0918-6158
- KRUEGER M ET AL: "Synthesis and stability of four maleimide derivatives of the anticancer drug doxorubicin for the preparation of chemoimmunoconjugates" CHEMICAL AND PHARMACEUTICAL BULLETIN, PHARMACEUTICAL SOCIETY OF JAPAN, TOKYO, JP, Bd. 45, Nr. 2, 1997, Seiten 399-401, XP002162561 ISSN: 0009-2363
- WILLNER D ET AL: "(6-MALEIMIDOCAPROYL)HYDRAZONE OF DOXORUBICIN - A NEW DERIVATIVE FORTHE PREPARATION OF IMMUNOCONJUGATES OF DOXORUBICIN" BIOCONJUGATE CHEMISTRY, ACS, WASHINGTON, DC, US, Bd. 4, 1993, Seiten 521-527, XP000783675 ISSN: 1043-1802
- BEYER U ET AL: "SYNTHESE VON NEUEN BIFUNKTIONELLEN MALEINIMIDVERBINDUNGEN ZUR HERSTELLUNG VON CHEMOIMMUNOKONJUGATEN SYNTHESIS OF NEW BIFUNCTIONAL MALCIMIDE COMPOUNDS FOR THE PREPARATION OF CHEMOIMMUNOCONJUGATES" MONATSHEFTE FUR CHEMIE, SPRINGER VERLAG. WIEN, AT, Bd. 128, 1997, Seiten 91-102, XP009023613 ISSN: 0026-9247

## Beschreibung

Nach dem Zusammenbringen mit dem Körper bindet die Substanz über das proteinbindende Molekül kovalent an Körperflüssigkeits- oder Gewebebestandteile, so daß eine Transportform des Wirkstoffs vorliegt, der pH-abhängig, hydrolytisch oder enzymatisch im Körper unter Freisetzung des Wirkstoffs spaltbar ist.

Der Großteil der zur Zeit eingesetzten Pharmaka sind niedermolekulare Verbindungen und weisen nach systemischer Applikation eine hohe Plasmasowie Gesamtclearance auf. Des weiteren dringen sie aufgrund von Diffusionsvorgängen in die Gewebestrukturen des Körpers ein und weisen in der Regel eine gleichmäßige Bioverteilung auf. Beide Eigenschaften führen dazu, daß nur geringe Mengen des Pharmakons den Wirkort erreichen und das Pharmakon aufgrund seiner Verteilung auf das gesunde Gewebe des Körpers Nebenwirkungen hervorruft. Diese Nachteile sind besonders bei solchen Pharmaka ausgeprägt, die ein hohes zytotoxisches Potential besitzen, wie etwa Zytostatika, Immunsuppressiva oder Virostatika.

Um die Selektivität von niedermolekularen Pharmaka zu verbessern, werden mehrere Strategien verfolgt, beispielsweise die chemische Derivatisierung von Leitstrukturen, die Formulierung als Prodrugs oder die Kopplung der Pharmaka an Trägermoleküle. Die vorliegende Erfindung geht von solchen Konzepten aus, bei denen Pharmaka an körpereigene Makromoleküle chemisch gebunden wurden. Bekannt sind Konjugate, bei denen im allgemeinen Zytostatika an Serumproteine, vorwiegend an bestimmte Trägermoleküle wie Humanserumalbumin und Humanserumtransferrin, gebunden werden und dann verabreicht werden. Diese bekannten Proteinkonjugate werden dadurch hergestellt, daß man ex vivo entweder in einem "Eintopfverfahren" das Zytostatikum an das Serumprotein koppelt (DE 41 22 210 A1) und das resultierende Albumin-Zytostatikum-Konjugat gewinnt oder daß man zunächst das Zytostatikum mit einem geeigneten Spacermolekül derivatisiert, das resultierende Produkt isoliert und in einem zweiten Schritt das so derivatisierte Zytostatikum über eine Maleinimidgruppe an das Protein koppelt (DE 196 36 889 A1 und PCT/DE 97/02000) und das resultierende Albumin-Zytostatikum-Konjugat isoliert. Beide Verfahren haben den Nachteil, daß Plasmaproteine verwendet werden, die pathogene Erreger enthalten können. Weitere Nachteile der beschriebenen Protein-Wirkstoff-Konjugate sind ihre unbefriedigende Stabilität und Lagerbeständigkeit und der technische Aufwand ihrer Herstellung.

Der Erfindung liegt die Aufgabe zugrunde, diese Nachteile zu überwinden. Gelöst wird diese Aufgabe durch Die Erfindung betrifft eine Substanz, die aus einem Wirkstoff, nämlich ein Zytostatikum, und mindestens einem kovalent proteinbindenden Molekülrest, nämlich eine Mafeinimidgruppe, der an den Wirkstoff durch einen Spacer gebunden ist, besteht,
worin der Spacer oder die Bindung zwischen dem Spacer und dem Wirkstoff pH-abhängig, hydrolytisch oder enzymatisch im Körper spaltbar ist, zur Verwendung als injizierbares Arzneimittel. Bei der Spaltung wird der Wirkstoff oder ein Derivat des Wirkstoffes freigesetzt. Unter einem Wirkstoffderivat werden Substanzen verstanden, die den Wirkstoff umfassen, jedoch zusätzlich Teile des Spacers oder der Gruppen, über die der Wirkstoff mit dem proteinbindenden Molekül gebunden war, enthalten können. Die Wirksamkeit des Wirkstoffs sollte durch seine Freisetzung als Derivat nicht beeinträchtigt werden. Vorzugsweise entfaltet der Wirkstoff bzw. dessen Derivat seine Wirksamkeit erst nach der Freisetzung.

Die Erfindung beruht auf der überraschenden Feststellung, daß es nicht, wie bisher angenommen wurde, nötig ist, einen Wirkstoff mit einem bestimmten Träger unter definierten Bedingungen zu verbinden und das Produkt zu verabreichen, sondern daß es möglich ist, therapeutisch und/oder diagnostisch wirksame Substanzen, bestehend aus einem pharmakologischen Wirkstoff, und wenigstens einem proteinbindenden Molekülteil, die durch einen Spacer miteinander verbunden sind, direkt als injizierbare Arzneimittel einzusetzen, da diese nach dem Zusammenbringen mit dem Körper über das proteinbindende Molekül kovalent derart an Körperflüssigkeits- oder Gewebebestandteile, vorwiegend an Serumproteine, binden, daß in vivo eine Transportform des Wirkstoffs gebildet wird, welche die Zielzellen bzw. das Zielgewebe des Wirkstoffs erreicht. Da erfindungsgemäß bei der therapeutisch wirksamen Substanz die Bindung im Spacermolekül oder zwischen dem Wirkstoff und dem Spacermolekül pH-abhängig, hydrolytisch oder enzymatisch im Körper spaltbar ist, wird der Wirkstoff trotzdem gezielt am gewünschten Zielort freigesetzt.

Erfindungsgemäß erhaltene Substanzen verändern und verbessern aufgrund ihrer proteinbindenden Eigenschaften das pharmakokinetische Profil der Wirkstoffe entscheidend. Wenn diese therapeutisch wirksame Substanzen in Körperflüssigkeiten gelangen, binden sie kovalent an Körperflüssigkeits- oder Gewebebestandteile, vorzugsweise an Serumproteine, mehr bevorzugt an Serumalbumin, um so als makromolekulare Prodrugs vorzuliegen, die den Wirkstoff zu dem Zielort transportieren und/oder ihn in einer dosierten Form freisetzen.

Die erfindungsgemäß erhaltene therapeutisch wirksame Substanz besteht aus einem Wirkstoff W, einem Spacermolekül SM und wenigstens einem proteinbindenden Molekül PM mit folgender allgemeiner Struktur:

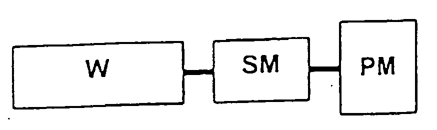

Zusätzlich kann die erhaltene Substanz Markierungsgruppen bzw. markierte Elemente oder Moleküle aufweisen, wobei sich die Substanz dann insbesondere für diagnostische Zwecke eignet. Bevorzugte Marker sind ein oder mehrere Radionuklide, ein oder mehrere Radionuklide umfassende Liganden, eine oder mehrere Positronenstrahler, ein oder mehrere NMR-Kontrastmittel, ein oder mehrere fluoreszierende Verbindung(en) oder/und ein oder mehrere Kontrastmittel im nahen IR-Bereich.

Ein Diagnostikum ist beispielsweise ein wie oben beschrieben markierter Wirkstoff oder ein oder mehrere fluoreszierende Verbindung(en) oder/und ein oder mehrere Kontrastmittel im nahen IR-Bereich.

Bevorzugte Zytostatika für die Herstellung von injizierbaren therapeutisch wirksamen Substanzen gemäß der vorliegenden Erfindung sind die Anthrazykline Doxorubicin, Daunorubicin, Epirubicin, Idarubicin, Mitoxantron und Ametantron sowie verwandte Derivate, die Alkylantien Chlorambucil, Bendamustin, Melphalan und Oxazaphoshorine sowie verwandte Derivate, die Antimetabolite Methotrexat, 5-Fluorouracil, 5 '-Desoxy-5-fluorouridin und Thioguanin sowie verwandte Derivate, die Taxane Paclitaxel und Docetaxel sowie verwandte Derivate, die Camptothecine Topotecan, Irinotecan, 9-Aminocamptothecin und Camptothecin sowie verwandte Derivate, die Podophyllotoxinderivate Etoposid, Teniposid und Mitopodozid sowie verwandte Derivate, die Vinca-Alkaloide Vinblastin, Vincristin, Vindesin und Vinorelbin sowie verwandte Derivate, die Calicheamicine, die Maytansinoide und eine Verbindung der allgemeinen Formel I bis XII: 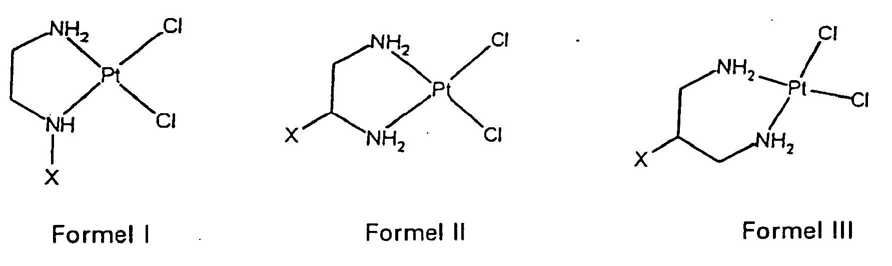 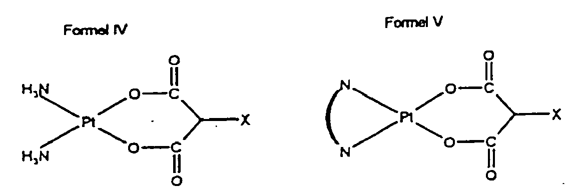 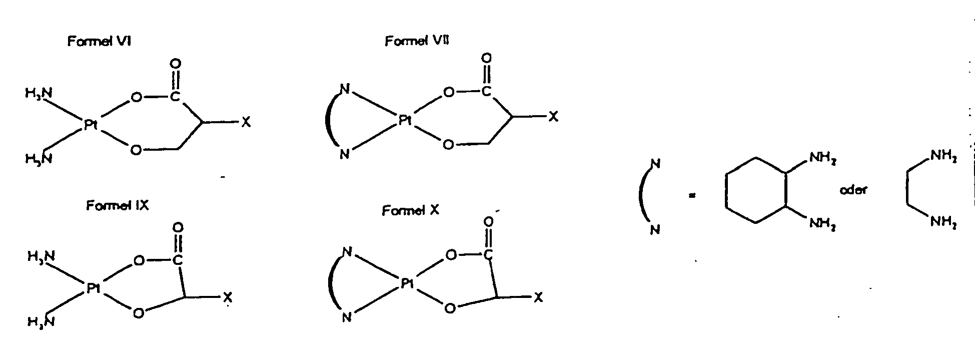 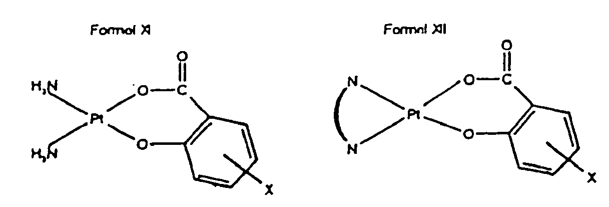 wobei X das Spacermolekül SM oder das proteinbindende Molekül PM bedeutet.

Das Spacermolekül SM ist ein organisches Molekül bestehend aus einer aliphatischen Kohlenstoffkette und/oder einem aliphatischen Kohlenstoffring und/oder mindestens einem Aromaten. Die/der aliphatische Kohlenstoffkette/-ring besteht vorzugsweise aus 1-12 Kohlenstoffatomen, die teilweise durch Sauerstoffatome ersetzt sein können, und kann gegebenenfalls substituiert sein, insbesondere durch eine oder mehrere wasserlösliche Gruppen, wie Sulfonsäure-, Aminoalkyl- oder Hydroxygruppen. Der Aromat ist vorzugsweise ein Benzolring, der gegebenenfalls substituiert sein kann, wie etwa mit den obengenannten wasserlöslichen Gruppen. Die aliphatische Kohlenstoffkette kann zur besseren Wasserlöslichkeit Sauerstoffatome enthalten und sich dazu zweckmäßig von einer Oligoethylenoxid- oder -propylenoxidkette ableiten, z.B. eine Diethylenglycol-, Triethylenglycol-oder Dipropylenglycolkette sein.

Das proteinbindende Molekül PM ist eine Maleinimidgruppe. Eine Halogenacetamidgruppe, eine Halogenacetatgruppe, eine Pyridyldithio-Gruppe, eine N-Hydroxysuccinimidester-oder eine Isothiocyanat-Gruppe werden als proteinbindendes Molekül beschrieben. Es kann auch eine Disulfidgruppe, eine Vinylcarbonylgruppe, eine Aziridingruppe oder eine Acetylengruppe sein. Die Disulfidgruppe kann aktiviert sein, in der Regel dadurch, daß eine Thionitrobenzoesäure (z.B. 5'-Thio-2-Nitrobenzoesäure) die austauschbare Gruppe darstellt. Die Gruppen können gegebenenfalls substituiert sein. Die Maleinimid-, kann gegebenenfalls mit Alkyl oder mit den obengenannten wasserlöslichen Gruppen substituiert sein. PM besitzt proteinbindende Eigenschaften, d.h. es bindet im physiologischen Milieu kovalent an bestimmte Aminosäuren auf der Proteinoberfläche. Dabei reagiert die Maleinimid-Gruppe, vorzugsweise mit HS-Gruppen von Cysteinen.

Die therapeutisch wirksame Substanz gelangt nach parenteraler Applikation in die Blutbahn und kann über PM an Proteine binden. Bevorzugt erfolgt die Bindung an Serumproteine, insbesondere Serumalbumin. Es wurde gefunden, daß im physiologischen Milieu die therapeutisch wirksame Substanz über die Maleinimid-Gruppe, insbesondere mit dem freien Cystein-34 des Albumins reagiert und auf diesem Weg kovalent gebunden wird. Serumproteine, wie Albumin oder Transferrin, weisen eine ausgesprochen lange Halbwertszeit im systemischen Kreislauf auf (bis zu 19 Tagen - Peters, T. Jr. (1985): Serum albumin. Adv. Protein. Chem. 37, 161-245). Aufgrund einer erhöhten Permeabilität von Gefäßwänden des malignen, infizierten bzw. entzündeten Gewebes für Makromoleküle gelangt Serumalbumin bevorzugt in dieses Zielgewebe (Maeda, H.; Matsumura, Y. Crit. Rev. Ther. Drug Carrier Sys. (1989), 6, 193-210). Dadurch kann ein an Albumin gekoppelter Wirkstoff gezielter den Wirkort erreichen. Des weiteren verhindert die kovalente Kopplung des Wirkstoffs an Serumproteine in der Blutbahn, daß der Wirkstoff in gesunde Gewebestrukturen des Körpers diffundiert oder über die Niere eliminiert wird bzw. diese in dem Maße schädigt wie der nicht gebundene Wirkstoff. Dadurch wird das pharmakokinetische Profil des Wirkstoffs verändert und verbessert, da seine Wirkung durch eine Anreicherung am Wirkort erhöht wird und gleichzeitig die toxischen Wirkungen auf gesunde Systeme des Körpers verringert werden.

Die gemäß der vorliegenden Erfindung verwendeten therapeutischwirksamen Substanzen enthalten im Spacermolekül oder zwischen SM und W eine definierte chemische Bindung. Diese Bindung ist pH-abhängig, vorzugsweise säurelabil, spaltbar bzw. hydrolytisch spaltbar, oder sie enthält mindestens eine Bindung, die im Körper enzymatisch gespalten wird, vorzugsweise eine Peptidbindung.

Bindungen, die durch Hydrolyse unter Freisetzung des Wirkstoffs gespalten werden, sind beispielsweise Esterbindungen oder Metallkomplexbindungen, wie sie bei Platin-Dicarboxylat-Komplexen vorliegen, wobei ein Diammindiaquo-Platin(II)-Komplex freigesetzt wird. Die säurelabilen spaltbaren Bindungen sind Acetal-, Ketal-, Imin-, Hydrazon, Carboxylhydrazon- oder Sulfonylhydrazonbindungen oder cis-Aconitylbindungen oder eine Tritylgruppe enthaltende Bindungen, wobei die Tritylgruppe substituiert oder nicht substituiert sein kann. Bevorzugte therapeutisch relevante säurelabile Bindungen sind beispielsweise in Kratz et al. (1990) Crit. Rev. Ther. Drug. Car.Sys. 16 (3), 245-288 beschrieben. Die Peptidsequenz in den realisierten Peptidbindungen besteht in der Regel aus etwa 2-30 Aminosäuren. Die Peptidsequenz ist dabei vorzugsweise auf die Substratspezifität bestimmter Enzyme, nachstehend als Targetenzyme bezeichnet, im Körper zugeschnitten, so daß die Peptidsequenz oder ein Teil diese Sequenz von einem Enzym im Körper erkannt wird und das Peptid gespalten wird. Gemäß einer weiteren Ausführungsform der vorliegenden Erfindung besteht die enzymatisch spaltbare Bindung aus einer Bindung, die keine Peptidbindung ist. Beispiele sind Carbamatbindungen, die durch krankheitsspezifische Enzyme, z.B. Glutathion-S-Transferasen, Glucuronidasen, Galactosidasen, den Wirkstoff oder ein Wirkstoffderivat freisetzen. Es ist auch ohne weiteres möglich, daß eine enzymatisch spaltbare Bindung aus einer Peptidsequenz und einer der obengenannten Bindungen, die keine Peptidbindung ist, aufgebaut ist. Die Targetenzyme können sowohl körpereigene Enzyme sein oder Enzyme, die in Mikroorganismen vorkommen bzw. von diesen gebildet werden.

Die Targetenzyme sind in der Regel Proteasen, Serinproteasen, Plasminogenaktivatoren und Peptidasen, beispielsweise Matrix-Metalloproteasen (MMP) oder Cysteinproteasen, die bei Erkrankungen wie rheumatoider Arthritis oder Krebs verstärkt gebildet oder aktiviert sind, was zum exzessiven Gewebeabbau, zu Entzündungen und zur Metastasierung führt. Targetenzyme sind insbesondere MMP 2, MMP3 und MMP 9, die als Proteasen bei den genannten pathologischen Prozessen beteiligt sind (Vassalli, J., Pepper, M.S. (1994), Nature 370, 14-15, Brown, P.D. (1995), Advan Enzyme Regul. 35, 291-301).

Weitere Proteasen, die Targetenzyme für therapeutisch wirksame Substanzen der vorliegenden Erfindung darstellen, sind Cathepsine, insbesondere Cathepsin B, H und L, die als Schlüsselenzyme bei entzündlichen und malignen Erkrankungen identifiziert worden sind.

Die oben genannten Bindungstypen gewährleisten, daß der Wirkstoff oder ein entsprechend aktives Derivat am Wirkort extrazellulär und/oder intrazellulär nach Aufnahme des Konjugats durch die Zelle freigesetzt wird und seine therapeutische entfalten kann.

Die Spaltung kann auch dergestalt ablaufen, dass nicht der Wirkstoff als solcher abgespalten wird, sondern ein Derivat des Wirkstoffs. Ein solches Derivat enthält somit den Wirkstoff sowie daran gebundene Gruppen, die aus dem Spacermolekül stammen, je nachdem, an welcher Stelle die gewünschte Spaltung erfolgt ist.

Die therapeutischwirksame Substanz kann gemäß einer der untenstehenden allgemeinen Beschreibungen hergestellt werden:

Wirkstoffe, die eine HOOC-Gruppe besitzen, werden folgendermaßen derivatisiert:

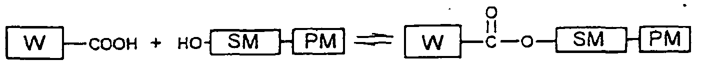

Die Veresterung erfolgt dabei durch übliche Verfahren, die dem Fachmann geläufig sind.

Es ist weiterhin möglich, die HOOC-Gruppe in eine Hydrazidgruppe zu überführen, z.B. durch Umsetzen mit tert.-Alkylcarbazaten und anschließende Spaltung mit Säuren (beschrieben in DE 196 36 889), und das eine Hydrazidgruppe aufweisende Pharmakon mit einem eine Carbonylkomponente enthaltenen Spacer, bestehend aus PM und SM, umzusetzen, wie u.a. in DE 196 36 889 A1 bzw. PCT/DE 97/02000 beschrieben ist: 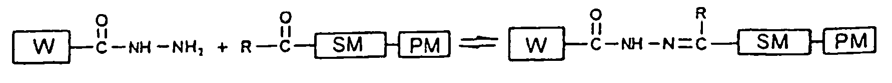 R = H, Alkyl, Phenyl, substituiertes Phenyl

Wirkstoffe der vorliegenden Erfindung, die eine H₂N-Gruppe besitzen, werden folgendermaßen derivatisiert: 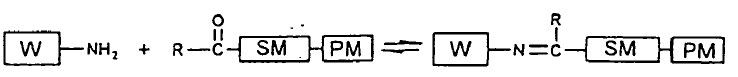 R = H, Alkyl, Phenyl, substituiertes Phenyl

Die Reaktion zu den Iminderivaten erfolgt dabei durch übliche Verfahren, die dem Fachmann geläufig sind.

Wirkstoffe der vorliegenden Erfindung, die eine HO-Gruppe besitzen, werden folgendermaßen derivatisiert:

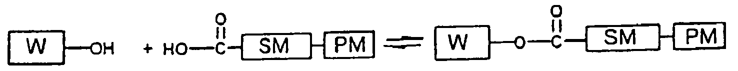

Die Veresterung erfolgt dabei durch übliche Verfahren, die dem Fachmann geläufig sind.

Wirkstoffe der vorliegenden Erfindung, die eine Carbonylkomponente besitzen, werden folgendermaßen derivatisiert: 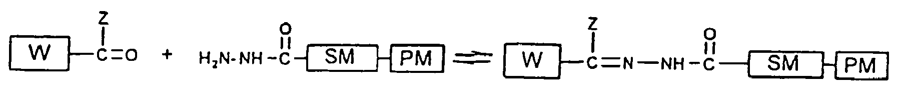 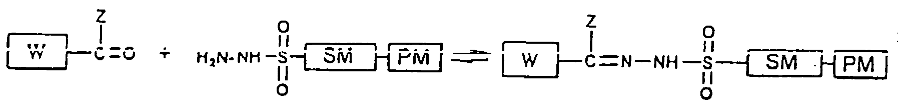 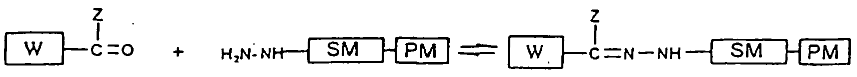 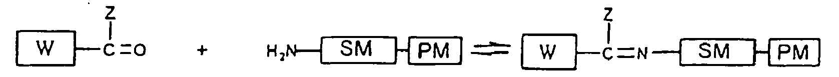 Z = chemische Gruppe des Wirkstoffs

Die Umsetzung zu den Carboxyhydrazon-, Sutfonythydrazon-, Hydrazon-bzw. Iminderivaten erfolgt dabei gemäß Verfahren, die u.a. in DE 196 36 889 A1 bzw. PCT/DE 97/02000 beschrieben sind, oder durch übliche Verfahren, die dem Fachmann geläufig sind.

Es ist weiterhin möglich, eine HO-Gruppe oder eine NH₂-Gruppe eines Wirkstoffs in eine Carbonylkomponente zu überführen, z.B. durch Veresterung bzw. Amidbildung mit einer Carbonsäure-tragenden Carbonylkomponente gemäß folgender allgemeiner Formel: 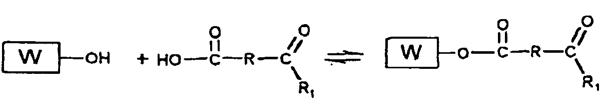 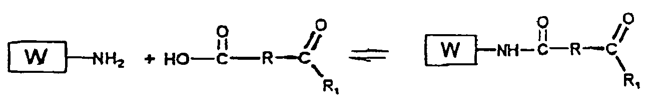 wobei R eine aliphatische Kohlenstoffkette und/oder ein aliphatischer Kohlenstoffring und/oder ein Aromat und R₁ = H, Alkyl-, Phenyl- oder eine substituierte Phenylgruppe ist. R besteht in der Regel aus 1 bis 12 Kohlenstoffatomen, die gegebenenfalls substituiert sein können, z.B. durch wasserlösliche Gruppen, wie Sulfonsäure-, Aminoalkyl- oder Hydroxygruppen. Der Aromat ist in der Regel ein Benzolring, der gegebenenfalls substituiert sein kann, wie etwa mit den oben genannten wasserlöslichen Gruppen.

Die Carbonylkomponente kann des Weiteren durch andere chemische Reaktionen eingeführt werden, so z.B. durch eine elektrophile Substitution an der HO- oder NH₂-Gruppe des Wirkstoffs mit einer geeigneten Carbonylkomponente.

Die so derivatisierten Wirkstoffe, die nun eine Carbonylkomponente besitzen, werden analog zu den oben beschriebenen Verfahren mit den proteinbindenden Spacermolekülen, die eine Amino-, Hydrazid- oder Hydrazingruppe besitzen, zu den entsprechenden Carboxylhydrazon-, Sulfonylhydrazon-, Hydrazon-bzw. Iminderivaten umgesetzt. Diesäurelabile Spaltung dieser Bindungen führt demnach zu einer Freisetzung des derivatisierten Wirkstoffs, der eine Carbonylkomponente besitzt.

Die Spacer, die aus dem proteinbindenden Molekül PM und dem Spacermolekül SM bestehen, können z.B. gemäß Verfahren, die u.a. in DE 196 36 889 A1, U. Beyer et al. Chemical Monthly, 128, 91, 1997**,** R.S. Greenfield et al, Cancer Res., 50, 6600, 1990, T. Kaneko et al., Bioconjugate Chem., 2, 133, 1991, Bioconjugate Techniques, G.T. Hermanson, Academic Press, 1996 oder in US-Patent 4,251,445 beschrieben sind, hergestellt werden.

Therapeutisch wirksame Substanzen für die vorliegende Erfindung, die eine Peptidbindung enthalten, können dadurch hergestellt werden, daß ein Peptid, das aus 2 bis etwa 30 Aminosäuren besteht, mit einer proteinbindenden Verbindung umgesetzt wird, so daß ein proteinbindendes Molekül direkt oder über ein Spacermolekül SM am N-terminalen Ende des Peptids eingeführt wird. Die Synthese von solchen proteinbindenden Peptidderivaten erfolgt vorzugsweise durch eine dem Fachmann geläufige Festphasensynthese, wobei im letzten Schritt des Peptidaufbaus ein Carbonsäure tragendes proteinbindendes Spacermolekül, z.B. eine Maleinimidocarbonsäure, durch Peptidkopplung an das N-terminale ende des Peptids gebunden und das proteinbindende Peptid im Anschluss von der Festphase abgespalten wird. Die so erhaltenen Peptidderivate können mit einem Wirkstoff, der eine H₂N- oder HO-Gruppe besitzt, in der Gegenwart eines Kondensationsmittels, wie zum Beispiel N,N'-Dicyclohexylcarbodiimid (DCC) oder N-Cyclohexyl-N'-(2-morpholinoethyl)-carbodiimid Metho-p-toluolsulfonat (CMC), (Benzotriazol-1-yloxy)-trispyrrolidinophosphoniumhexafluorophosphat (pyBOP) oder O-Benzotriazol-N,N,N',N'-tetramethyluroniumhexafluorophosphat und gegebenenfalls unter Zusatz von N-Hydroxysuccinimid oder eines wasserlöslichen N-Hydroxysuccinimids, wie etwa N-Hydroxysuccinimid-3-sulfonsäure Natriumsalz oder 1-Hydroxybenzotriazol und/oder in Gegenwart einer Base, wie z.B. N-Methylmorpholin oder Triethylamin, zu den entsprechenden proteinbindenden Wirkstoff-Peptidderivaten umgesetzt werden:

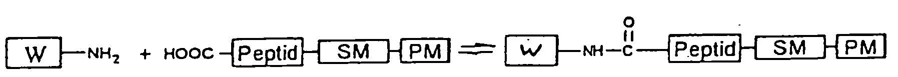

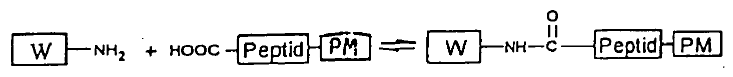

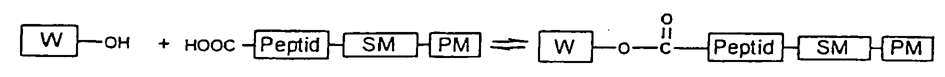

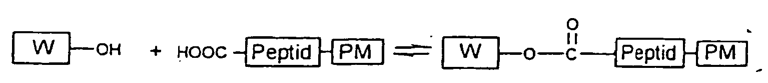

Es ist weiterhin möglich, über die HOOC-Gruppe der Wirkstoffe eine H₂N-oder HO-Gruppe einzuführen, beispielsweise durch Derivatisierung mit den Aminosäuren (AS) Lysin, Serin oder Threonin über deren α-Aminogruppe oder über die α-Aminogruppe mit einer Diaminoverbindung der allgemeinen Formel H₂N―(CH₂)ₙ―NH₂ oder mit einem Alkoholamin der allgemeinen Formel H₂N―(CH₂)ₙ―OH mit n = 1-12, und diese Derivate im Anschluß mit den oben genannten Peptidderivaten zu den entsprechenden proteinbindenden Wirkstoff-Peptidderivaten umzusetzen: 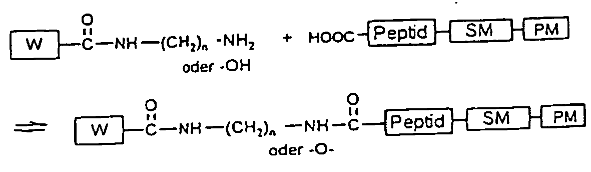 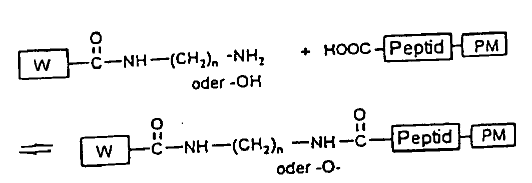 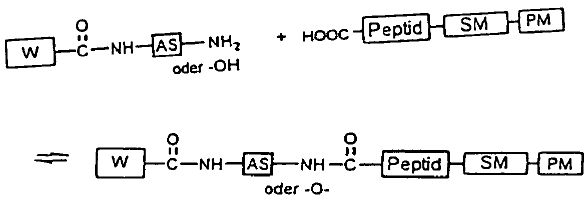 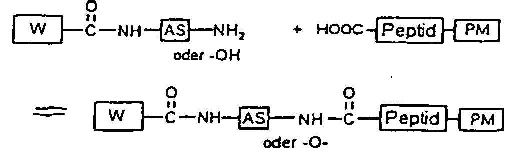 AS = Lysin, Serin oder Threonin

Die Substratspezifität von Targetenzymen, wie etwa von MMP 2, MMP3, MMP 9, Cathepsin B, H und L, ist bekannt (Netzel-Arnett et al. (1993), Biochemistry 32, 6427-6432, Shuja, S., Sheahan, K., Murname, M.J. (1991), Int.J. Cancer 49, 341-346, Lah, T.T., Kos, J. (1998), Biol. Chem. 379, 125-130).

Beispielsweise sind Octapeptide (P₄ - P'₄) für MMP 2 und MMP 9 identifiziert worden, welche die Spaltsequenz der Kollagenkette simulieren, und besonders effizient von MMP 2 und 9 gespalten werden:

| Peptid | | | | | | | |
|---|---|---|---|---|---|---|---|
| P₄ | P₃ | P₂ | P₁ | P'₁ | P'₂ | P'₃ | P'₄ |

Gly-Pro-Leu-Gly- Ile-Ala-Gly-Gln
Gly-Pro-Gln-Gly-Ile-**Trp**-Gly-Gln
(Netzel-Arnett et al., Biochemistry 32, 1993, 6427-6432).

Die Peptide werden ausschließlich an der P₁ - P'₁-Bindung enzymatisch gespalten.

Desweiteren sind bei Cathepsin B substratspezifische Dipeptide bekannt mit der Sequenz -Arg-Arg-, -Phe-Lys-, Gly-Phe-Leu-Gly, Gly-Phe-Ala-Leu oder Ala-Leu-Ala-Leu (Werle, B., Ebert, E., Klein, W., Spiess, E. (1995), Biol. Chem. Hoppe-Seyler 376, 157-164; Ulricht, B., Spiess, E., Schwartz-Albiez, R., Ebert, W. (1995), Biol. Chem. Hoppe-Seyler 376, 404-414).

Die Peptidsequenz, welche die für das Targetenzym relevante Peptidsollbruchstelle enthält, kann auch so aufgebaut sein, daß die Peptidsollbruchstelle mehrfach wiederholt wird, wie beispielsweise durch:
-Gly-Pro-Leu-Gly-lie-Ala-Gly-Gln-Gly-Pro-Leu-Gly-Ile-Ala-Gly-Gln
   oder
-Phe-Lys-Phe-Lys-Phe-Lys-Phe-Lys-Phe-Lys-Phe-Lys-
   oder es kann eine repetitive Peptidsequenz integriert werden, die den Abstand zwischen dem proteinbindenden Molekül und der relevanten Peptidsollbruchstelle vergrößert, wie beispielsweise durch:
-Gly-Gly-Gly-Gly-Gly-Gly-Gly-Gly-Gly-Gly-Gly-Gly-Phe-Lys-Phe-Lys-

Entscheidend für die therapeutisch wirksamen Substanzen zur Verwendung in der vorliegenden Erfindung ist die Tatsache, daß die für das jeweilige Targetenzym relevante Peptidsollbruchstelle mindestens einmal in einem Oligopeptid vorkommt. Die oben aufgeführten Oligopeptide sind repräsentative Beispiele für die Herstellung von therapeutisch wirksame Substanzen.

Therapeutisch wirksame Substanzen, die ein Zytokin enthalten, können dadurch hergestellt werden, daß das Zytokin mit einem eine proteinbindende Gruppe enthaltenen Spacermolekül, das eine Carbonsäure oder eine aktivierte Carbonsäure aufweist, umgesetzt wird:

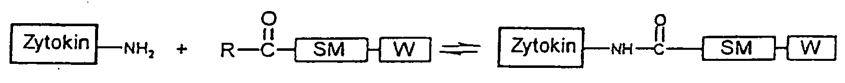

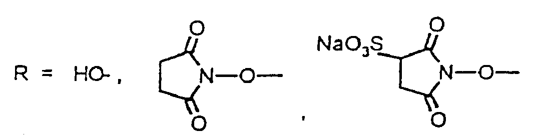

Weist das Spacermolekül eine N-Hydroxysuccinimidester-Gruppe (N-Hydroxysuccinimid oder N-Hydroxysuccinimid-3-sulfonsäure Natriumsalz) auf, wird es direkt mit dem Zytokin umgesetzt. Die Umsetzung des Zytokins mit einem eine proteinbindenden Gruppe enthaltenen Spacermolekül, das eine Carbonsäure aufweist, erfolgt in der Gegenwart eines Kondensationsmittels, wie zum Beispiel. N,N'-Dicyclohexylcarbodiimid (DCC) oder N-Cyclohexyl-N'-(2-morpholinoethyl)-carbodiimid Metho-p-toluolsulfonat (CMC), und gegebenenfalls unter Zusatz von N-Hydroxysuccinimid oder N-Hydroxysuccinimid-3-sulfonsäure Natriumsalz, zu den entsprechenden proteinbindenden Zytokinderivaten. Die Aufreinigung der so derivatisierten Zytokine erfolgt zweckmäßig mit Hilfe der Ausschlußchromatographie. Die oben beschriebenen Umsetzungen sind dem Fachmann geläufig (Bioconjugate Techniques, G.T. Hermanson, Academic Press, 1996).

Im Anschluß an die Synthese der therapeutisch wirksamen Substanz wird eine injizierbare Arzneimittelzubereitung, enthaltend die therapeutisch wirksame Substanz, in einer geeigneten Trägerflüssigkeit hergestellt. Die therapeutisch wirksame Substanz liegt bevorzugt als Lyophilisat vor, wobei vor oder nach dem Lyophilisieren übliche Träger und/oder pharmazeutische Hilfsstoffe, wie etwa Polysorbate, Glucose, Lactose, Mannose, Citronensäure, Tromethamol, Triethanolamin oder Aminoessigsäure beigefügt sein können. Die injizierbare Arzneimittelzubereitung muß so hergestellt werden, daß das proteinbindende Molekül durch das Lösen in der injizierbaren Trägerflüssigkeit nicht deaktiviert, abgespalten oder hydrolysiert wird. Weiterhin muß gewährleistet werden, daß die säurelabile Bindung in der therapeutisch wirksamen Substanz, die eine Ester-, Acetal-, Ketal-, Imin-, Hydrazon, Carboxylhydrazon- oder Sulfonylhydrazonbindung ist, nicht hydrolysiert wird. Die im Rahmen der vorliegenden Erfindung verwendeten proteinbindenden Moleküle sind basenempfindlich, so daß der pH-Wert der Trägerflüssigkeit einen pH-Wert von 8,0 nicht überschreiten soll. Bevorzugt liegt der pH-Wert im Bereich von pH 4,0-7,0, mehr bevorzugt zwischen pH 6,0 und pH 7,0. Außerdem muß die Trägerflüssigkeit natürlich physiologisch verträglich sein.

Bevorzugte Trägerflüssigkeiten sind annähernd isotonische Salzpuffer, z.B. Phosphat-, Acetat- oder Citratpuffer, wie etwa, 0.004 M Natriumphosphat, 0.15 M NaCl - pH 6,0-7,0 oder 0.01 M Natriumacetat, 0.14 M NaCl - pH 5,0-6,5). Die verwendete Trägerflüssigkeit kann auch eine isotonische Natriumchloridlösung sein. Die Salzpuffer können übliche Träger und/oder Hilfsstoffe, wie etwa Polysorbate, Glucose, Lactose, Mannose, Citronensäure, Tromethamol, Triethanolamin oder Aminoessigsäure enthalten.

Die Löslichkeit der therapeutisch wirksamen Substanz in der injizierbaren Trägerflüssigkeit kann durch pharmazeutische Lösungsmittel, wie etwa Ethanol, Isopropanol, 1,2-Propylenglykol, Glycerol, Macrogole, Polyethylenglykole bzw. Polyethylenoxide oder durch Lösungsvermittler, z.B. Tween, Cremophor oder Polyvinylpyrrolidon, verbessert werden. Zu diesem Zweck wird die therapeutisch wirksame Substanz entweder in dem pharmazeutischen Lösungsmittel bzw. Lösungsvermittler gelöst und anschließend mit einem Salzpuffer verdünnt oder eine Trägerflüssigkeit, enthaltend den Salzpuffer und mindestens ein pharmazeutisches Lösungsmittel bzw. Lösungsvermittler, wird zum Lösen der therapeutisch wirksamen Substanz direkt verwendet. Die Konzentration der pharmazeutischen Lösungsmittel bzw. Lösungsvermittler überschreiten dabei nicht die Mengen, die vom Arzneimittelgesetz (AMG) vorgeschrieben sind.

Vorzugsweise sollte die Trägerflüssigkeit so ausgewählt werden, daß der Lösevorgang der therapeutisch wirksamen Substanz in der Trägerflüssigkeit nach wenigen Minuten abgeschlossen ist, so daß eine injizierbare Arzneimittelzubereitung am Krankenbett zur Verfügung gestellt wird.

Die therapeutisch wirksame Substanz eignet sich somit zur Behandlung von Krebskrankheiten, Viruskrankheiten, Autoimmunerkrankungen, akuten oder chronisch-entzündlichen Erkrankungen und/oder Erkrankungen, die durch Bakterien, Pilze oder andere Mikroorganismen verursacht werden.

Ferner wird eine diagnostisch wirksame Substanz mit Gehalt an wenigstens einem Diagnostikum, die dadurch gekennzeichnet ist, daß sie wenigstens einen proteinbindenden Molekülrest, welcher mit dem Diagnostikum durch einen Spacer verbunden ist, wobei der Spacer oder die Bindung zwischen Spacer und Diagnostikum pH-abhängig, hydrolytisch oder enzymatisch im Körper spaltbar ist unter Freisetzung des Diagnostikums beschrieben. Das Diagnostikum kann ein oder mehrere Readionuklide, ein oder mehrere Radionuklide umfassende Liganden, ein oder mehrere Positronenstrahler, ein oder mehrere NMR-Kontrastmittel, ein oder mehrere fluoreszierende Verbindung(en) oder/und ein oder mehrere Kontrastmittel im nahen IR-Bereich umfassen.

Ferner wird ein diagnostischer Kit beschrieben, enthaltend eine proteinbindende diagnostisch wirksame Substanz, gegebenenfalls zusammen mit dem Trägermolekül und pharmazeutisch annehmbaren Hilfsstoffen, Trägerstoffen und/oder Verdünnungsmitteln, insbesondere ausgewählt aus den oben genannten. Der diagnostische Kit kann zum Nachweis der wie oben definierten Erkrankungen oder zum Nachweis von Trägermolekülen und/oder deren Verteilung im Organismus verwendet werden.

Es wird ein Verfahren zur Herstellung einer injizierbaren Arzneimittelzubereitung, enthaltend eine diagnostisch wirksame Substanz, die in einer injizierbaren Trägerflüssigkeit gelöst wird beschrieben, welches dadurch gekennzeichnet ist, dass als diagnostisch wirksame Substanz eine Verbindung umfassend ein Diagnostikum und wenigstens einen proteinbindenden Molekülrest verwendet wird. Ein Diagnostikum kann eine der oben genannten Verbindungen sein, beispielsweise ein oder mehrere Radionuklide, ein oder mehrere Radionuklide umfassende Liganden, ein oder mehrer Positronenstrahler, ein oder mehrere NMR-Kontrastmittel, ein oder mehrere fluoreszierende Verbindung(en) oder/und ein oder mehrere Kontrastmittel im nahen IR-Bereich. Das Diagnostikum und der wenigstens eine proteinbindende Molekülrest können auch durch einen Spacer verbunden sein. Hierbei ist es bevorzugt, dass der Spacer oder die Bindung, welche die beiden Komponenten verbindet, nicht spaltbar ist. Beispiele für nicht im Körper spaltbare Bindungen, die bei der Bindung zur diagnostisch wirksamen Substanz vorliegen können, sind Amidbindungen, gesättigte bzw. ungesättigte Kohlenstoff-Kohlenstoff-Bindungen oder Bindungen zwischen Kohlenstoff und einem Heteroatom, -C-X-, wobei X vorzugsweise O, N, S oder P ist. Eine Bindung ist beispielsweise eine Amidbindung. Die Freisetzung der therapeutisch wirksamen Substanz ist bevorzugt, da in der Regel der niedermolekulare Wirkstoff mit seinem molekularen Target wechselwirken muß, um seine pharmakologische Wirksamkeit zu entfalten. Bei diagnostisch wirksamen Substanzen hingegen ist eine Freisetzung des Protein gebundenen Diagnostikums nicht unbedingt erforderlich, kann aber vorhanden sein. Die diagnostisch wirksame Substanz kann zusätzlich über eine nicht im Körper spaltbare Bindung an das Spacermolekül oder direkt an die proteinbindende Gruppe ohne Vorhandensein eines Spacermoleküls SM gebunden sein.

Die folgenden Beispiele erläutern die Erfindung näher.

### Beispiel 1

### Herstellung von DOXO-HYD

Die im folgenden abgebildete pharmakologisch aktive Substanz (abgekürzt DOXO-HYD) besteht aus dem Zytostatikum Doxorubicin, aus einer Maleinimidgruppe als proteinbindendes Molekül PM und aus einem Phenylacetylhydrazonspacer als Spacermolekül SM. Die Bindung zwischen Doxorubicin und dem Spacermolekül SM ist eine säurelabile Carboxylhydrazonbindung:

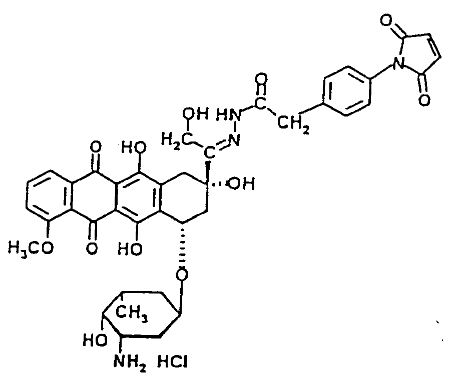

10,51 mg DOXO-HYD werden in 2,0 ml 1,2-Propylenglykol durch Schütteln gelöst und anschließend wird diese Lösung mit 8,0 ml Phosphatpuffer (0.004 M Natriumphosphat, 0.15 M NaCl - pH 6,5) verdünnt und homogenisiert (Konzentration von DOXO-HYD in der Trägerflüssigkeit ≈ 1300 *µ*M). Die so hergestellte injizierbare Arzneimittelzubereitung von DOXO-HYD wurde Versuchstieren unmittelbar intravenös verabreicht (siehe unten).

### Beispiel 2

### Bindung von DOXO-HYD an Humanplasma

Nachdem DOXO-HYD in die Blutbahn gelangt, findet eine Bindung an Serumproteine statt, vorzugsweise an Serumalbumin, so daß DOXO-HYD u.a. als säurelabiles Albumin-Doxorubicin-Konjugat vorliegt.

Inkubationsstudien von Humanblutplasma mit DOXO-HYD bei 37 °C zeigen, daß nach einer 5-minütigen Inkubation der Großteil von DOX-HYD kovalent an Albumin gebunden ist. **(A)** - im Gegensatz zu freiem Doxorubicin **(B)**. Dieser Sachverhalt ist in den untenstehenden Chromatogrammen dargestellt. (**A** und **B**):

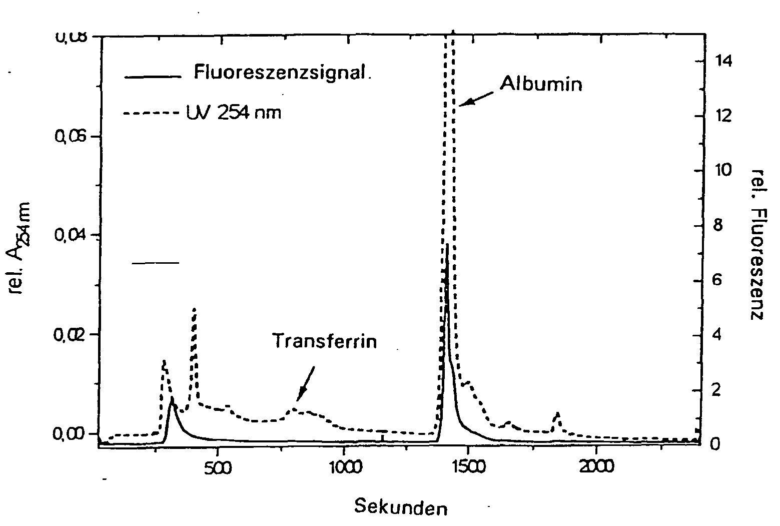

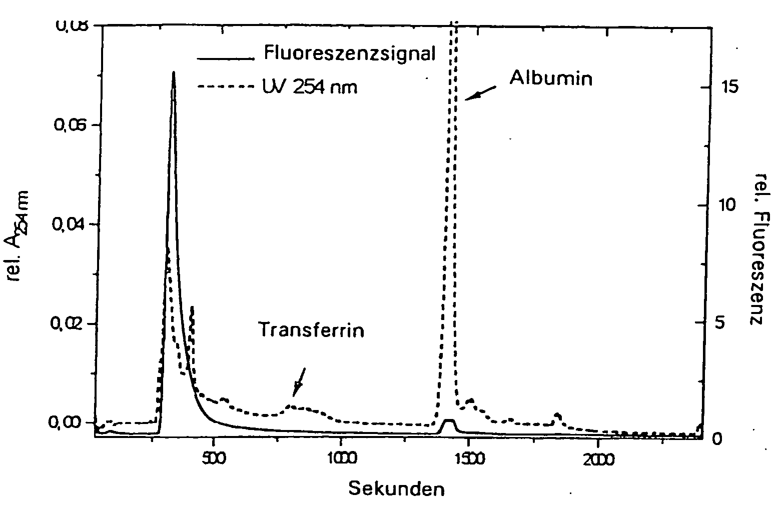

Hierbei wurde nach erfolgter Inkubation die Plasmaprobe über eine POROS®⁻20-Anionenaustauschersäule getrennt (Detektion der Proteine bei 254 nm, Detektion des Anthrazyklins durch Fluoreszenz).

### Beispiel 3

### Die Wirksamkeit von DOXO-HYD in vivo

Die unten aufgeführten biologischen Daten verdeutlichen die in vivo Wirksamkeit von DOXO-HYD im Vergleich zu freiem Doxorubicin: Im sogenannten RENCA (renal cell carcinoma)-Modell wurde Doxorubicin und DOXO-HYD hinsichtlich der antitumoralen Wirksamkeit bei annähernd äquitoxischer Dosis miteinander verglichen (intravenöse Therapie 10 Tage nach Injektion von etwa 1 Million Nierenkarzinomzellen in der linken Niere).

**Tiere:** Balb/c-Mäuse, weiblich; **Tumor:** RENCA, renal cell carcinoma (Nierenzellkarzinom)
**Therapie:** Tag(d) 10, 13, 17 und 20 intravenöse (i.v.), Ende des Versuchs d24

| Anzahl der Mäuse | Substanz | Dosis | Durchschnittliche Körpergewichtsabnahme (%) d 1-24 |
|---|---|---|---|
| | | | |
| 10 | Kontrolle | | |
| 10 | Doxorubicin | 4 x 10 mmol/kg | -15 |
| 10 | DOXO-HYD | 4 x 20 mmol/kg | -15 |
| | | | |

Die Ergebnisse dieses Versuches sind untenstehend abgebildet. DOXO-HYD zeigt eine sehr gute antitumorale Wirksamkeit und erzielt eine deutliche Reduktion des Nierentumorvolumens und der Anzahl von Lungenmetastasen im Vergleich zur Kontrollgruppe und der Doxorubicin behandelten Gruppe.

### Gewicht und Volumen der Nieren und Nierentumoren sowie Anzahl der Lungenmetastasen

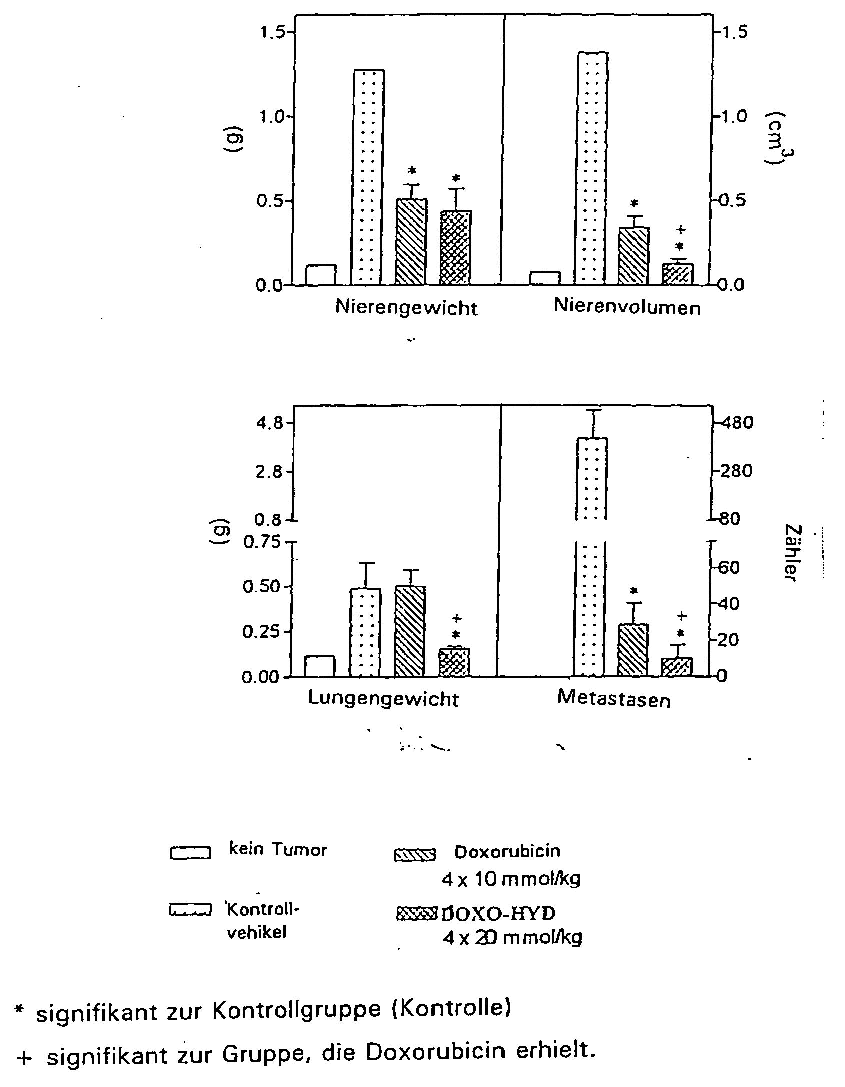

### Beispiel 4

### Bindung von DOXO-EMHC an Albumin im Humanplasma

1,6 mg des 6-Maleinimidocapronsäurehydrazon-Derivats von Doxorubicin (abgekürzt DOXO-EMHC) mit untenstehender Strukturformel 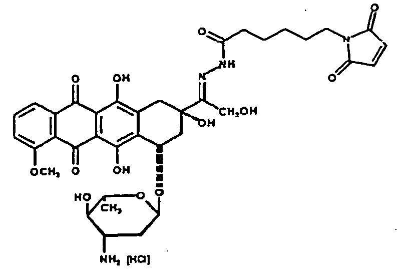 werden in 1,0 ml Phosphatpuffer (0,15 M NaCl, 0,004 M Natriumphosphat, pH 6,5) bei Raumtemperatur gelöst (2000 *µ*M Lösung). Werden 250 *µ*l dieser Lösung mit 1,0 ml Humanplasma 30 Sekunden lang bei 37 °C inkubiert, und die Probe anschließend über einen schwachen Anionenaustauscher (von POROS® ) getrennt, zeigt sich, daß der überwiegende Teil von DOXO-EMHC an Albumin gebunden ist (s. untenstehendes Chromatogramm):

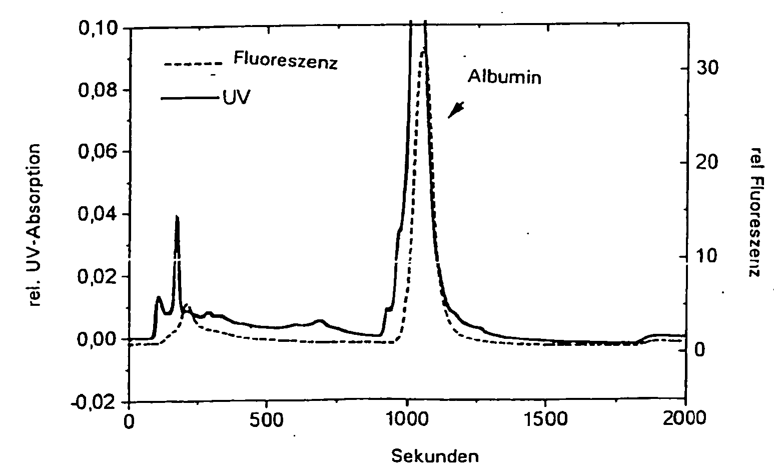

### Beispiel 5

### Bindung eines durch MMP9 spaltbares Doxorubicin-Maleinimid-Peptid-Derivats (2) an Albumin nach einminütiger Inkubation mit Humanplasma

Das Doxorubicin-Maleinimid-Peptid-Derivat (2) wurde gemäß folgender Reaktionsgleichung hergestellt:

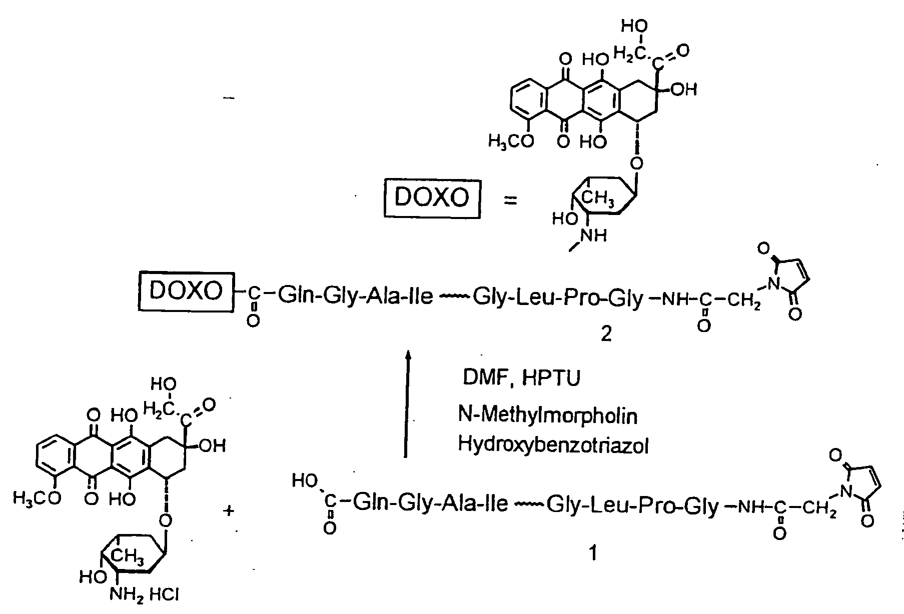

Dabei wird das mit Maleinimidoglycin derivatisierte Octapeptid Gln-Gly-Ala-Ile-Gly-Leu-Pro-Gly 1 (Mr 848, hergestellt durch Festphasensynthese durch Bachem AG, Schweiz) mit Doxorubicin laut folgender Vorschrift umgesetzt:

Zu einer leicht trüben Lösung von 17,1 mg Doxorubicin in 3 ml DMF werden 25 mg 1 (als Trifluoracetatsalz) gelöst in 500 *µ*l DMF, 33,5 mg O-Benzotriazol-N,N,N',N'-tetramethyluroniumhexafluorphosphat (HPTU) gelöst in 200 *µ*l DMF, 11,9 mg Hydroxybenzotriazolhydrat gelöst in 100 *µ*l DMF und 16,2 *µ*l N-Methylmorpholin zugegeben, und der Ansatz anschließend 18 h lang bei RT im Dunklen gerührt. DMF wurde am Hochvakuum entfernt und der Feststoff in 20 ml Methanol aufgenommen, filtriert und im Vakuum auf 1 ml eingeengt. Nach Aufreinigung über Kieselgel (Essigester/Methanol 2/1) wurden 5 mg 2 erhalten.

### Inkubationsstudie mit Humanplasma

1,4 mg 2 (Mr 1374) werden in 1,0 ml Phosphatpuffer (0,15 M NaCl, 0,004 M Natriumphosphat, pH 6,5) bei Raumtemperatur gelöst (1000 *µ*m Lösung). Werden 300 *µ*l dieser Lösung mit 1,0 ml Humanplasma 60 Sekunden lang bei 37 °C inkubiert, und die Probe anschließend über einen schwachen Anionenaustauscher (von POROS® ) getrennt, zeigt sich, daß der überwiegende Teil von 2 an Albumin gebunden ist (s. untenstehendes Chromatogramm):

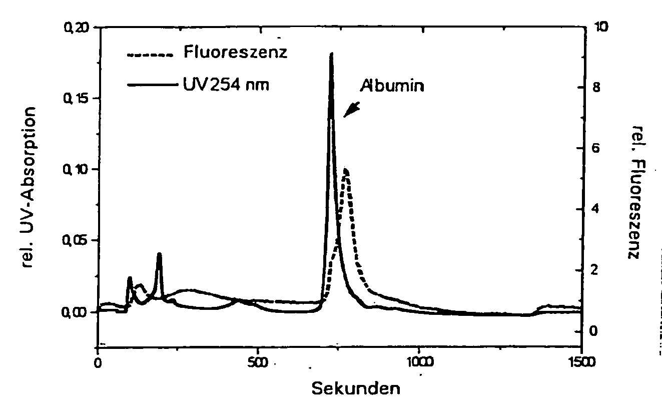

Die Peptidsequenz Gln-Gly-Ala-Ile-Gly-Leu-Pro-Gly wird von der Matrixmetalloprotease MMP9 erkannt und zwischen Isoleucin und Glycin gespalten. Dies wurde durch folgenden Versuch gezeigt: 200 *µ*l einer 100 *µ*M Lösung des Albuminkonjugats von 2 mit folgender Struktur (abgekürzt HSA-2): 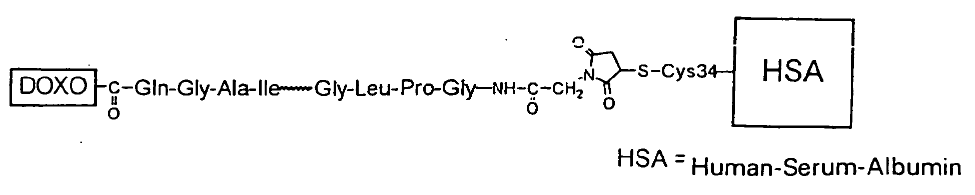 das durch das in der deutschen Patentanmeldung A19926475.9 vom 10. Juni 1999 beschriebene Verfahren hergestellt wurde, wurde mit Trypsin/Aprotinin aktivierter MMP9 (2 mU von Calbiochem, Deutschland) 30 Minuten lang bei 37 °C inkubiert. Die Freisetzung von DOXO-Gln-Gly-Ala-Ile nach dieser Zeit ist in den untenstehenden Chromatogrammen abgebildet. Gezeigt ist das Chromatogramm von HSA-2 bei t = 0 (Trennung durch HPLC Ausschlußchromatographie mit einer Biosil 250 SEC Säule von der Firma Biorad, Detektion bei λ = 495 nm) und nach einer Inkubationszeit mit aktivierter MMP9 von 30 Minuten.

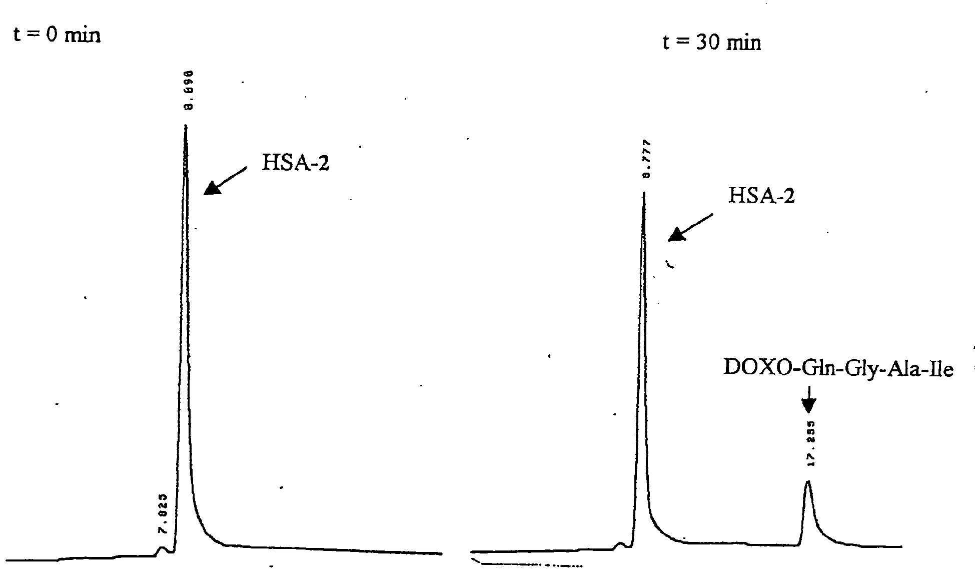

### Vergleichs Beispiel 6

### Bindung von Fluoresceinmaleinimid an Albumin im Humanplasma

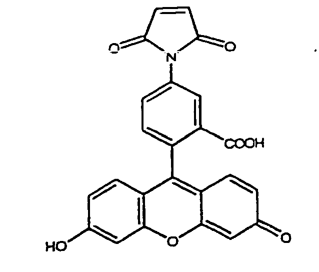

Nach einer 5-minütigen Inkubation von 250 *µ*l einer 100 *µ*M Fluoresceinmaleinimid-Lösung (Phosphatpuffer - 0,15 M NaCl, 0,004 M Natriumphosphat, pH 5,0) mit 1,0 ml Humanplasma und anschließenden Trennung der Probe mittels der Ausschlußchromatographie (Superdex® 200, Pharamcia), zeigt sich, daß der überwiegende Teil des Fluoresceinmaleinimids an Albumin gebunden ist (s. untenstehendes Chromatogramm):

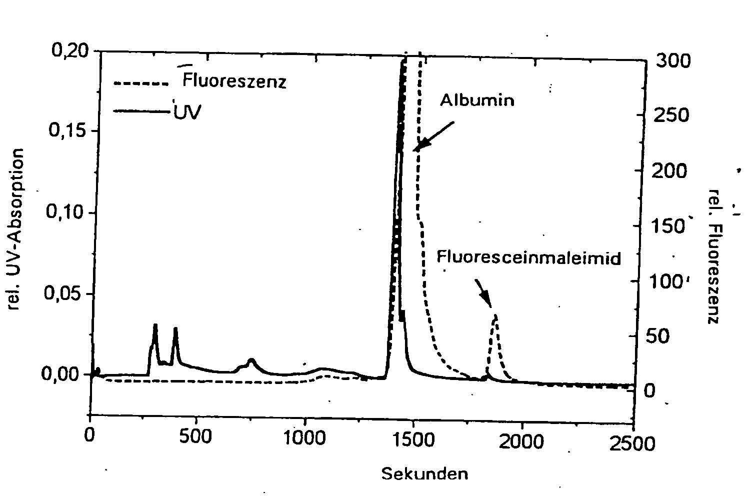

## Patentansprüche

1. Substanz, die aus
einem Wirkstoff, nämlich ein Zytostatikum, und mindestens einem kovalent proteinbindenden Molekülrest, nämlich eine Maleinimidgruppe, der an den Wirkstoff durch einen Spacer gebunden ist, besteht,
worin der Spacer oder die Bindung zwischen dem Spacer und dem Wirkstoff pH-abhängig, hydrolytisch oder enzymatisch im Körper spaltbar ist, zur Verwendung als injizierbares Azneimittel.

2. Substanz, die aus
einem Wirkstoff, nämlich ein Zytostatikum, und mindestens einem kovalent ρroteinbindenden Molekülrest, nämlich eine Maleinimidgruppe, der an den Wirkstoff durch einen Spacer gebunden ist, besteht,
worin der Spacer oder die Bindung zwischen dem Spacer und dem Wirkstoff pH-abhängig, hydrolytisch oder enzymatisch im Körper spaltbar ist, und
worin der proteinbindende Molekülrest nicht an ein Trägerprotein gebunden ist, zur Verwendung als injizierbares Arzneimittel.

3. Substanz nach einem der Ansprüche 1-2,
**dadurch gekennzeichnet,**
**dass** die Substanz die chemische Formel: 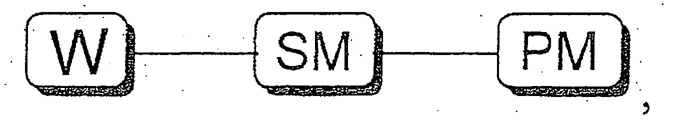 aufweist,
worin
W der Wirkstoff ist;
SM ein Spacermolekül ist und
PM ein proteinbindendes Molekül ist.

4. Substanz nach Anspruch 3,
**dadurch gekennzeichnet,**
**dass** die Substanz die chemische Formel: 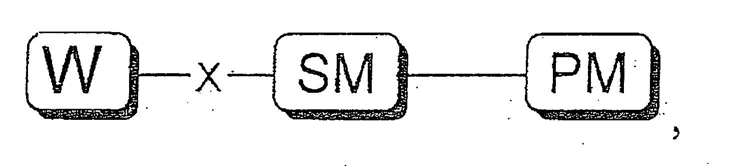 aufweist, worin
X eine chemische Gruppe ist, ausgewählt aus: 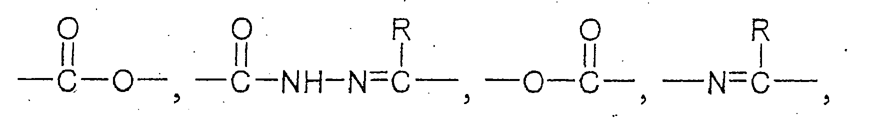 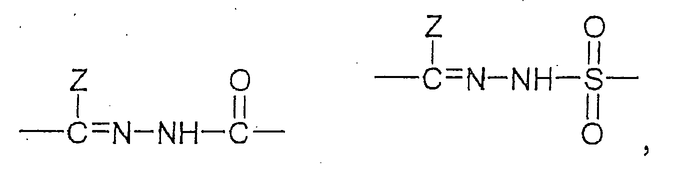 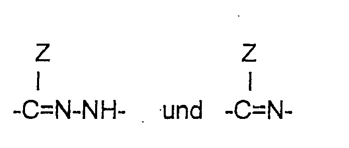 worin
R = H, Alkyl, Phenyl oder substituiertes Phenyl, und
Z = chemische Gruppe des Wirkstoffs.

5. Substanz nach Anspruch 4,
**dadurch gekennzeichnet,**
**dass** die Substanz die chemische Formel: 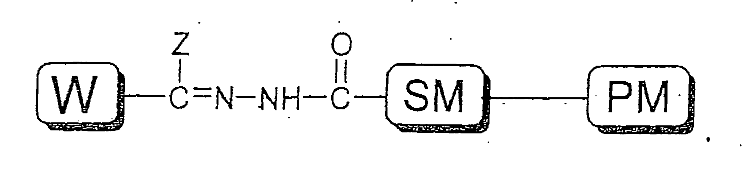 aufweist.

6. Substanz nach einem der Ansprüche 1-5,
**dadurch gekennzeichnet,**
**dass** der Wirkstoff aus der Gruppe der Anthrazykline, der Stickstofflostderivate, der Alkylantien, der Purin- oder Pyrimidinantagonisten, der Folsäureantagonisten, der Taxane, der Camptothecine, der Podophyllotoxinderivate, der Vinca-Alkaloide, der Calicheamicine, der Maytansinoide oder der cis-konfigurierten Platin(II)-Komplexe ausgewählt ist.

7. Substanz nach Anspruch 6,
**dadurch gekennzeichnet,**
**dass** das Anthracyclin Doxorubicin, Daunorubicin, Epirubicin, Idarubicin, Mitoxantron, oder Ametantron und insbesondere Doxorubicin umfaßt.

8. Substanz nach einem der Ansprüche 1-7,
**dadurch gekennzeichnet,**
**dass** das Spacermolekül ein organischer Molekülrest ist, welcher eine aliphatische Kohlenstoffkette und/oder einen aliphatischen Kohlenstoffring mit 1-12 Kohlenstoffatomen, die teilweise durch Sauerstoffatome ersetzt sein können, und/oder mindestens einen Aromaten enthält, die ggf. substituiert sein können.

9. Substanz nach einem der Ansprüche 1-8,
**dadurch gekennzeichnet,**
**dass** die Substanz 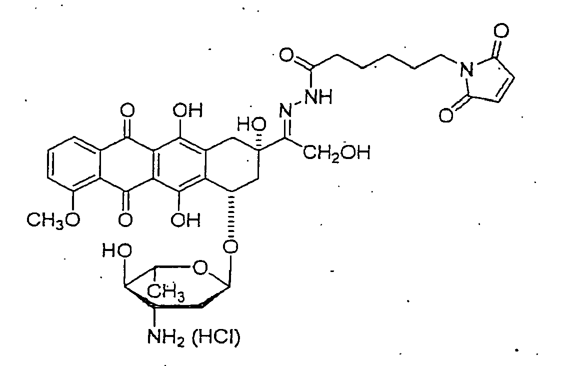 ist.

10. Substanz nach einem der Ansprüche 1-9 zur Verwendung als Arzneimittel zur Behandlung von Krebskrankheiten, Viruskrankheiten, Autoimmunerkrankungen, akuten oder chronisch-entzündlichen Erkrankungen oder Erkrankungen, die durch Bakterien, Pilze oder andere Mikroorganismen verursacht sind.

11. Zubereitung umfassend eine Substanz nach einem der Ansprüche 1-10 und eine Trägerflüssigkeit.

## Claims

1. Substance which consists of an active substance, namely a cytostatic agent and at least one covalently protein-binding molecular residue namely a maleinimide group which is bound to the active substance by a spacer, wherein the spacer or the bond between the spacer and the active substance can be pH-dependently, hydrolytically or enzymatically cleaved in the body, for use as an injectable pharmaceutical preparation.

2. Substance which consists of an active substance, namely a cytostatic agent and at least one covalently protein-binding molecular residue, namely a maleinimide group which is bound to the active substance by a spacer, wherein the spacer or the bond between the spacer and the active substance can be pH-dependently, hydrolytically or enzymatically cleaved in the body, and
wherein the protein-binding molecular residue is not bound to a carrier protein, for use as an injectable pharmaceutical preparation.

3. Substance according to one of the claims 1 - 2,
**characterized in that**
the substance has the chemical formula: 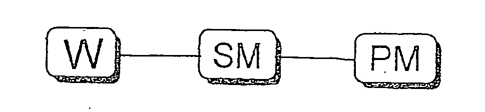 wherein,
W is the active substance,
SM is a spacer molecule and
PM is a protein-binding molecule.

4. Substance according to claim 3,
**characterized in that**
the substance has the chemical formula: 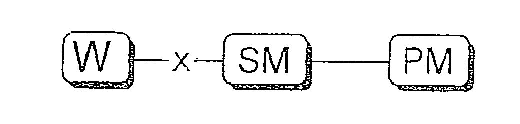 wherein
X is a chemical group selected from: 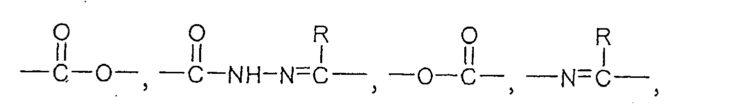 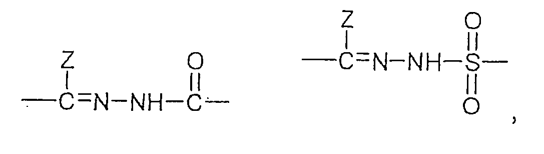 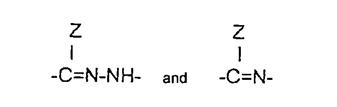 wherein
R = H, alkyl, phenyl or substituted phenyl and
Z = a chemical group of the active substance.

5. Substance according to claim 4,
**characterized in that**
the substance has the chemical formula:

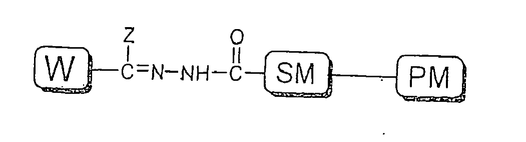

6. Substance according to one of the claims 1 - 5
**characterized in that**
the active substance is selected from the group of anthrocyclines, nitrogen mustard derivatives, alkylating agents, purine or pyrimidine antagonists, folic acid antagonists, taxanes, camptothecines, podophyllotoxin derivatives, vinca alkaloids, calicheamicins, maytansinoids or cis-configured platinum (II) complexes.

7. Substance according to claim 6,
**characterized in that**
the anthracycline comprises doxorubicin, daunorubicin, epirubicin, idarubicin, mitoxantrone or ametantrone and in particular doxorubicin.

8. Substance according to one of the claims 1 - 7,
**characterized in that**
the spacer molecule is an organic molecular residue which contains an aliphatic carbon chain and/or an aliphatic carbon ring with 1 - 12 carbon atoms which can be partially replaced by oxygen atoms, and/or at least one aromatic compound, which can be optionally substituted.

9. Substance according to one of the claims 1 - 8,
**characterized in that**
the substance is

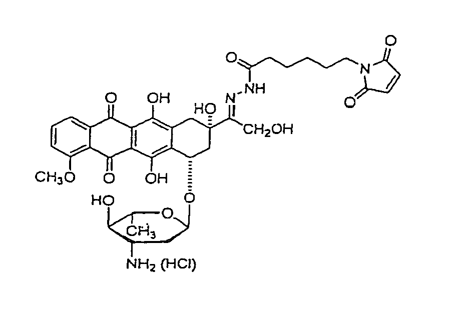

10. Substance according to one of the claims 1 - 9 for use as pharmaceutical preparations for treating cancer diseases, viral diseases, autoimmune diseases, acute or chronic inflammatory diseases or diseases which are caused by bacteria, fungi or other microorganisms.

11. Preparation comprising a substance according to one of the claims 1 - 10 and a carrier liquid.

## Revendications

1. Substance qui consiste en
une substance active, à savoir un cytostatique, et
en au moins un résidu moléculaire se liant de façon covalente à une protéine, à savoir un groupe maléinimide qui est lié à la substance active par un espaceur,
dans laquelle l'espaceur ou la liaison entre l'espaceur et la substance active peut être clivé(e) de façon pH-dépendante, hydrolytique ou enzymatique dans le corps, pour l'utilisation en tant que médicament injectable.

2. Substance qui consiste en
une substance active, à savoir un cytostatique, et
en au moins un résidu moléculaire se liant de façon covalente à une protéine, à savoir un groupe maléinimide qui est lié à la substance active par un espaceur,
dans laquelle l'espaceur ou la liaison entre l'espaceur et la substance active peut être clivé(e) de façon pH-dépendante, hydrolytique ou enzymatique dans le corps, et dans laquelle le résidu moléculaire se liant à une protéine n'est pas lié à une protéine de support, pour l'utilisation en tant que médicament injectable.

3. Substance selon l'une des revendications 1 à 2,
**caractérisée en ce**
**que** la substance présente la formule chimique : 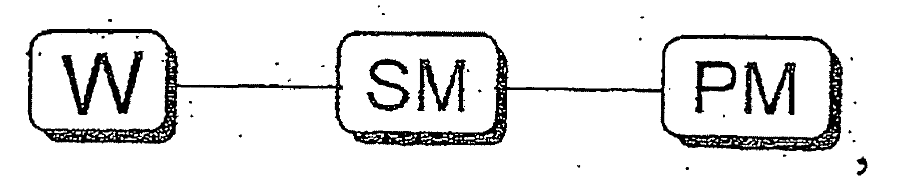 dans laquelle
W est la substance active
SM est une molécule d'espaceur et
PM est une molécule se liant à la protéine.

4. Substance selon la revendication 3,
**caractérisée en ce**
**que** la substance présente la formule chimique : 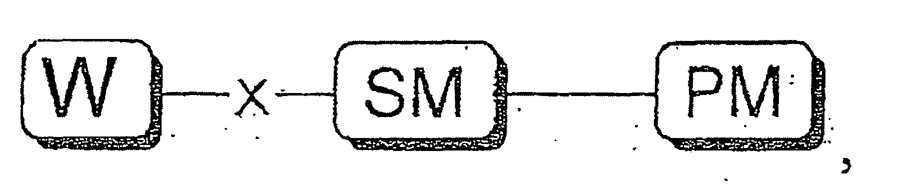 dans laquelle
X est un groupe chimique choisi parmi : 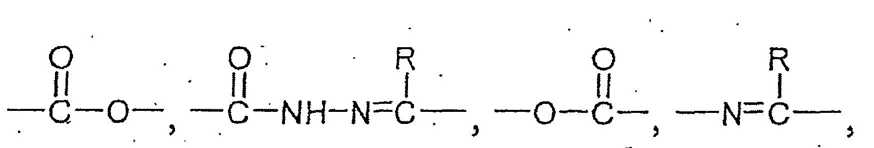 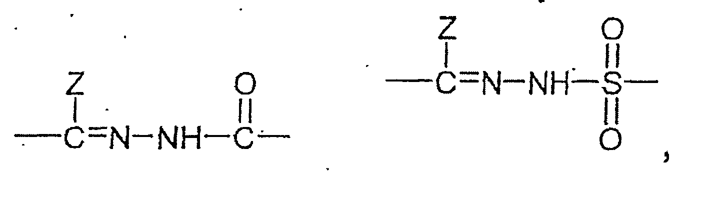 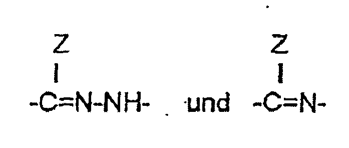 dans laquelle
R = H, un groupe alkyle, phényle ou phényle substitué, et
Z = un groupe chimique de la substance active.

5. Substance selon la revendication 4,
**caractérisée en ce**
**que** la substance présente la formule chimique :

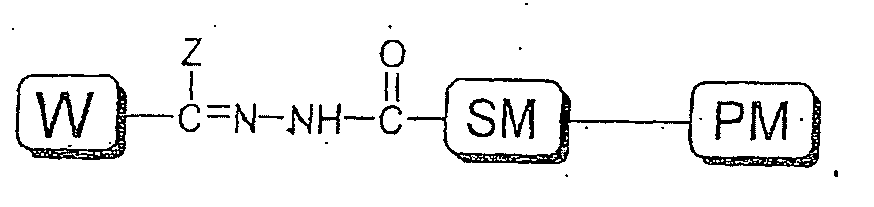

6. Substance selon l'une des revendications 1 à 5,
**caractérisée en ce**
**que** la substance active est choisie dans le groupe comprenant les anthracyclines, les dérivés d'ypérite azotée, les alkylants, les antagonistes de la purine ou de la pyrimidine, les antagonistes de l'acide folique, les taxanes, les camptothécines, les dérivés de podophylotoxine, les alcaloïdes vinca, les calicheamicines, les maytansinoïdes ou les complexes de platine (II) à configuration cis.

7. Substance selon la revendication 6,
**caractérisée en ce**
**que** l'anthracycline comprend la doxorubicine, la daunorubicine, l'épirubicine, l'idarubicine, la mitoxantrone ou l'amétantrone et en particulier la doxorubicine.

8. Substance selon l'une des revendications 1 à 7,
**caractérisée en ce**
**que** la molécule espaceur est un radical moléculaire organique qui contient une chaîne de carbone aliphatique et/ou un cycle de carbone aliphatique, comportant 1 à 12 atomes de carbone qui peuvent, le cas échéant, être partiellement substitués.

9. Substance selon l'une des revendications 1 à 8,
**caractérisée en ce**
**que** la substance est

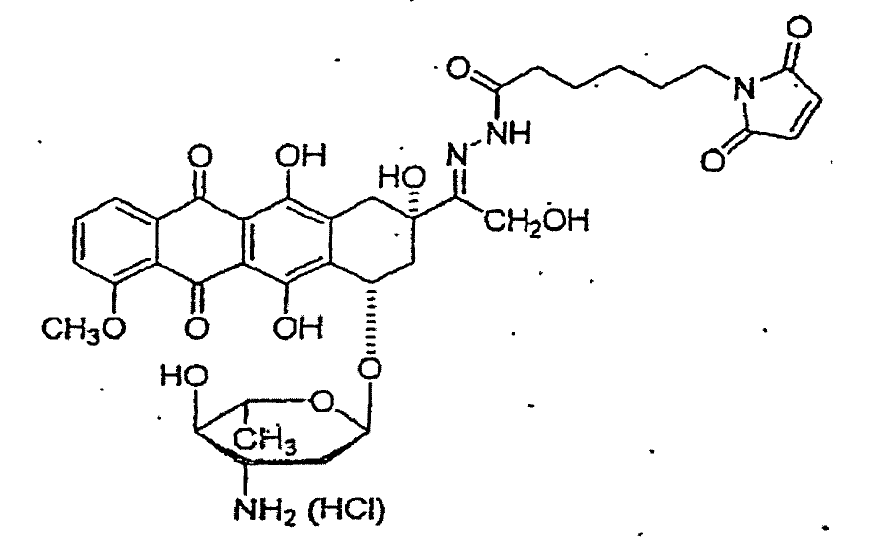

10. Substance selon l'une des revendications 1 à 9, à utiliser comme médicament pour le traitement des cancers, des maladies virales, des maladies auto-immunes, des maladies inflammatoires aiguës ou chroniques ou des maladies qui sont provoquées par des bactéries, des champignons ou d'autres microorganismes.

11. Préparation comprenant une substance selon l'une des revendications 1 à 10 et un liquide de support.
